# EUROPÄISCHE PATENTANMELDUNG

(11) **EP 2 401 992 A1**
(43) Veröffentlichungstag der Anmeldung: **04.01.2012**
(21) Anmeldenummer: 11170322.9
(22) Anmeldetag: 17.06.2011
(51) Int. Cl.: A61F 13/02

(54) **Verband**

(30) Priorität: 02.07.2010 DE 202010007967 U
(71) Anmelder: McAirlaid's Vliesstoffe GmbH & Co. KG, 37339 Berlingerode (DE)
(72) Erfinder: Schmidt, Andreas, 37115 Duderstadt (DE); Söder, Jens, 37081 Göttingen (DE)
(74) Vertreter: Christophersen, Ulrich Rudolf

(57) **Zusammenfassung**

Es wird ein Verband zum Abdecken einer Körperöffnung, aus welcher ein nicht-körpereigener Strang, Schlauch, Leitung oder dgl. herausragt, beansprucht, der einen Träger (8) mit einem vom Rand des Trägers (8) ausgehenden und in den Träger (8) hineinragenden Schlitz (5) unter Bildung einer ersten Trägerhälfte (7a) zur einen und einer zweiten Trägerhälfte (7b) zur anderen Seite des Schlitzes (5), wobei auf jeder der beiden Trägerhälften (7a, 7b) in paralleler Ausrichtung zum Schlitz (5) jeweils ein flächiger Saugkern (3, 4) angebracht ist, und dadurch gekennzeichnet, dass der Schlitz (5) im Bereich seines Endes mit einer Erweiterung (6) versehen ist. Der Verband kann beispielswiese als Schlitzkompresse eingesetzt werden.

## Beschreibung

Die vorliegende Erfindung betrifft einen Verband zum Abdecken einer Körperöffnung, aus welcher eine Leitung herausragt, welche umfasst einen Träger mit einem Schlitz unter Bildung einer ersten Trägerhälfte und einer zweiten Trägerhälfte, wobei auf jeder der beiden Trägerhälften in paralleler Ausrichtung zum Schlitz jeweils ein flächiger Saugkern angebracht ist.

Verbände und insbesondere Kompressen sind seit langer Zeit zur Behandlung von akuten Wunden und chirurgisch angelegten Körperöffnungen, aus denen Blut, Wundsekret und andere Körperflüssigkeiten austreten, in der Notfallmedizin oder zur Verwendung bei chirurgischen Eingriffen bekannt. Diese Verbände dienen dazu, die Wunden bzw. Körperöffnungen abzudecken, um das Eindringen von Fremdkörpern in die Wunde zu verhindern und Blut und Wundsekret aufzunehmen.

Für das Abdecken von Körperöffnungen, die chirurgisch angelegt sind, um Körperflüssigkeit über eine Kanüle oder Leitung abzuleiten, werden häufig Schlitzkompressen verwendet. Diese Schlitzkompressen bestehen in der Regel aus einem rechteckigen Vlies oder anderem saugfähigen Material, welches einen etwa bis zur Mitte des Materials hin reichenden Schlitz aufweist. Durch den Schlitz haben derartige Kompressen den Vorteil, dass ein Anlegen und Entfernen oder Wechseln der Kompresse möglich ist, ohne dass die Kanüle oder Leitung entfernt werden muss. Aus dem Stand der Technik sind vielfältige Schutzkompressen bekannt. So gibt es einstückig gebildete Kompressen, die ausschließlich aus saugfähigem Material bestehen. Diese Kompressen haben den Nachteil, dass sie aufgrund des relativ dicken saugfähigen Materials sehr fest sind, was sich für den Patienten nachteilig auf den Tragekomfort auswirkt. Auch ist der Materialbedarf an saugfähigem Vlies hoch, was die Materialkosten für derartige Verbände erhöht.

In der WO 2007/118664 wird ein Verband zur Umlage um eine chirurgisch angelegte Leitung aus einer Körperöffnung oder -höhle (Schlitzkompresse) offenbart, der wenigstens eine flüssigkeitsabsorbierende Matte mit einem Schlitz aufweist, wobei die flüssigkeitsabsorbierende Matte von einer Hülle umgeben ist, die eine Barriere für feste grobe Ausscheidungen bildet und die die Aufnahme bzw. das Aufsaugen von austretenden und/oder entfernungspflichtigen Substanzen durch den innerhalb der Höhle angeordneten Absorptionskörper ermöglicht. Die Matte ist ferner von wenigstens einer Schaumstofflage unterlegt.

Die aus dem Stand der Technik bekannten Kompressen haben den Nachteil, dass das Absorptionsmaterial einstückig geformt ist, wodurch die Kompresse selbst sehr fest ist und der Tragekomfort nicht optimal ist. Des Weiteren ist der Materialverbrauch an Absorptionsmaterial sehr hoch. Der Schlitz hat den Nachteil, dass Kanülen oder Leitungen mit einem größeren Querschnitt nicht optimal umschlossen werden können; es bilden sich Wölbungen, so dass die Kompresse nicht voll auf der abzudeckenden Fläche anliegt.

Der vorliegenden Erfindung lag somit die Aufgabe zugrunde, einen Verband, insbesondere eine Schlitzkompresse zur Verfügung zu stellen, die die aus dem Stand der Technik bekannten Nachteile nicht aufweist, insbesondere sollte der Tragekomfort für den Patienten verbessert und die Materialkosten verringert werden.

Gegenstand der vorliegenden Erfindung ist demgemäß ein Verband zum Abdecken einer Körperöffnung, aus welcher ein nicht-körpereigener Strang, Schlauch, Leitung oder dgl. herausragt, welcher einen Träger mit einem vom Rand des Träger ausgehenden und in den Träger hineinragenden Schlitz unter Bildung einer ersten Trägerhälfte zur einen einer zweiten Trägerhälfte zur anderen Seite des Schlitzes, wobei auf jeder der beiden Trägerhälften in paralleler Ausrichtung zum Schlitz jeweils ein flächiger Saugkern angebracht ist, dadurch gekennzeichnet, dass der Schlitz im Bereich seines Endes mit einer Erweiterung versehen ist.

Im Folgenden wird der nicht-körpereigene Strang, Schlauch, Leitung und dgl. kurz als Leitung bezeichnet.

Der Verband gemäß der vorliegenden Erfindung hat den Vorteil, dass er zum Abdecken von Wunden oder chirurgisch angelegten Körperöffnungen auch um nicht körpereigene Leitungen und dgl. herum gelegt werden kann, ohne dass ein Wechseln dieser Leitungen erforderlich ist. Auch ein Wechseln des Verbandes ist möglich, ohne dass die nicht-körpereigene Leitung gewechselt werden muss. Diese Leitung wird von der Erweiterung im Bereich des Endes des Schlitzes umschlossen, auch die Ränder des Schlitzes liegen somit im Wesentlichen glatt auf der zu verbindenden Körperoberfläche auf. Ein weiterer Vorteil ist, dass das eigentliche die Körperflüssigkeit aufnehmende Absorptionsmaterial, ein flächiger Saugkern, in Form von zwei voneinander getrennten Materialabschnitten auf einem Träger aufgebracht ist. Das Trägermaterial in Verlängerung zum Schlitz weist in der Regel keinen Saugkern auf, wodurch der Verband als Ganzes flexibler ist, was zu einem höheren Komfort für den Patienten führt.

Es hat sich als geeignet erwiesen, dass die Erweiterung im Bereich des Schlitzendes ist und von größerer Breite als der Schlitz ist. Die Größe dieser Öffnung sollte an die üblicherweise für Körperflüssigkeiten ableitende Leitungen, wie sie in der Chirurgie und im Pflegebereich Anwendung finden, angepasst sein. Für die Gestaltung der Erweiterung im Bereich des Schlitzendes gibt es eine Vielzahl von unterschiedlichen Ausgestaltungsmöglichkeiten. Die Erweiterung kann z. B. in der Art einer Schlüsselloch-Erweiterung ausgestaltet sein, die rund, dreieckig, viereckig oder auch mehreckig sein kann. In einer möglichen weiteren Ausgestaltung ist das Schlitzende in Form eines Kreuzes ausgestaltet. In einer weiteren möglichen Ausgestaltung ist das Schlitzende mit einer sich quer erstreckenden Erweiterung versehen, sodass der Schlitz die Form eines "T" aufweist, wobei die sich quer erstreckende Erweiterung gerade sein oder einen Bogen beschreiben kann.

Der Saugkern bzw. die Saugkerne sind erfindungsgemäß auf einem Träger angebracht. Üblicherweise liegt der Verband mit der Trägerseite auf der Körperoberfläche des Patienten auf. Das Trägermaterial sollte derart ausgestaltet sein, dass es das Eindringen von Fremdkörpern in die Öffnung von außen verhindert und eine solche Struktur aufweisen, dass die Körperflüssigkeit über das Trägermaterial zum Saugkern diffundiert und vom Saugkern absorbiert werden kann. Als Materialien für den Träger haben sich insbesondere Nonwoven (Vliese) und Gewebe aus natürlichen oder synthetischen Fasern und/oder gelochte zweiund/oder dreidimensionale Folien erwiesen. Diese Materialien sind dem Fachmann aus dem Bereich der Wundversorgung bekannt.

In einer bevorzugten Ausführungsform weist der flächige Saugkern auch auf der dem Träger abgewandten Flachseite einen weiteren Träger auf. Dieser weitere Träger weist vorzugsweise die gleiche Form auf wie der erste Träger. Einer oder beide Träger erstrecken sich vorzugsweise über die Fläche des Saugkerns hinaus und sind an dem Überstand miteinander verbunden, so dass der flächige Saugkern von den beiden Trägerlagen quasi umhüllt ist. Die beiden Trägerlagen sind an ihrem Überstand vorzugsweise miteinander verklebt, sie können auch verschweißt oder in sonstiger an sich bekannter Weise miteinander verbunden sein. Der erste Träger und der weitere Träger können aus gleichen oder aus unterschiedlichen Materialien bestehen, vorzugsweise bestehen beide Träger aus Nonwoven. Das Nonwoven (Vlies) hat den Vorteil, dass es austretende Körperflüssigkeit gut von der Körperöffnung zum Saugkern transportieren kann. Das der Körperoberfläche zugewandte Trägermaterial kann auch außerhalb der Körperöffnung ganz oder teilweise mit einem Haftmittel beschichtet sein, so dass der Verband ohne zusätzliche Hilfe auf der Körperoberfläche befestigt oder fixiert wird.

In einer möglichen Ausgestaltung der vorliegenden Erfindung sind die flächigen Saugkerne an den gegenüberliegenden Flachseiten von einem Trägermaterial mit Schlitz bedeckt. Vorzugsweise ist das Trägermaterial der beiden Träger in den Bereichen, in denen die beiden Träger direkt aneinanderstoßen durch Verkleben oder in sonstiger Weise miteinander verbunden. Diese Verbindung hat insbesondere den Vorteil, dass die austretende Körperflüssigkeit über beide Trägerschichten zum Saugkern transportiert werden kann. Die Saugleistung des erfindungsgemäßen Verbandes kann weiter gesteigert werden, wenn der Saugkern auf seiner Flachseite mit dem Träger bzw. den Trägern direkt verbunden ist, beispielsweise verklebt ist. Durch diese Verbindung erfolgt eine besonders schnelle Aufnahme der Körperflüssigkeit durch den Saugkern, der Strom der aufgenommenen Flüssigkeit wird nicht durch einen Zwischenraum zwischen Träger und Saugkern unterbrochen. Des Weiteren wird durch diese Verbindung die Stabilität des erfindungsgemäßen Verbandes erhöht.

Der flächige Saugkern kann aus einem beliebigen Absorptionsmaterial sein, wie Zellulose und/oder einem anderen Material mit hoher Absorptionskapazität. Der Saugkern dient dazu, die aus der Körperöffnung ausgetretenen Exsudate aufzunehmen und dauerhaft zu absorbieren. Eine Rücknässung in Richtung Körperöffnung sollte vorzugsweise weitgehend vermieden werden. Der Saugkern wird daher vorzugsweise von einem Material gebildet, welches dazu in der Lage ist, Körperflüssigkeiten nicht nur zu absorbieren, sondern auch zu speichern, wie Zellstoff oder Zellstoff-verwandte Materialien sowie synthetische saugfähige Materialien. Der Saugkern ist vorzugsweise ein Vlies aus Zellstofffasern wie ein Airlaid.

Vorzugsweise wird ein Airlaid aus Zellstofffasern als Absorptionsmaterial eingesetzt. In einer bevorzugten Ausgestaltung der vorliegenden Erfindung weist der Saugkern über seine Fläche verteilte Prägebereiche, in denen die Fasern, vorzugsweise Zellstofffasern, stärker als in den übrigen Bereichen miteinander verpresst und hierdurch klebstoff- und/oder bindemittelfrei verbunden sind.

In einer Ausführungsform ist der Saugkern aus einer Faserstoffbahn aus Zellstofffasern hergestellt, welche unter Erzeugung eines Prägemusters im Druckbereich bindemittelfrei, punkt- oder linienförmig kalandriert und verbunden sind. Die Herstellung derartiger Materialien ist beispielsweise im europäischen Patent 1 032 342 beschrieben.

Der Saugkern kann die Prägebereiche an der der Haut zugewandten Fläche und/oder der Haut abgewandten Fläche aufweisen. Die Faserschicht des Saugkerns ist demnach so strukturiert, dass die Zellstofffasern außerhalb dieser diskreten Prägebereiche gelockert übereinander oder nur schwach aneinander haftend vorliegen, wohingegen sie in den Prägebereichen miteinander verpresst sind und eine innige Verbindung mit den jeweils benachbarten Zellstofffasern eingehen. Durch diese Ausgestaltung ist ein völliger Verzicht auf Klebstoffe und Bindemittel zur Bildung des Verbundes aus Zellstofffasern möglich, was ein einfaches und vollständiges Recycling ermöglicht. In den Prägebereichen haften die Fasern nicht lediglich aneinander. Vielmehr wird durch die Druckbeaufschlagung erreicht, dass benachbarte Zellstofffasern in diesen Prägebereichen fest miteinander verbunden sind. Diese Verbindung vermag auch der Einwirkung durch Feuchtigkeit zu widerstehen, so dass sich der erfindungsgemäße Absorptionskörper durch mechanische Belastbarkeit auch in nassem Zustand auszeichnet.

Die Bereiche außerhalb der diskreten Prägebereiche, in denen die Fasern gelockert übereinander oder nur schwach einander haftend vorliegen, zeichnet sich durch eine gute Absorptionsfähigkeit aus. Die absorbierte Flüssigkeit wird von den Fasern aufgenommen und über die gesamte Fläche des Saugkerns verteilt und dort gehalten.

Um die Absorptionsfähigkeit des Saugkerns weiter zu steigern, kann dieser Teilchen aus superabsorbierenden Polymeren enthalten. Diese können beispielsweise direkt bei der Herstellung des Saugkerns mit eingearbeitet werden.

Die vorliegende Erfindung wird anhand der beigefügten Figuren näher erläutert. Es zeigen:
- Figur 1: eine Draufsicht auf den Verband,
- Figur 2: eine Schnittdarstellung entlang der Linie A-A,
- Figur 3: in vergrößerter Darstellung einen Schnitt durch den Saugkern,
- Figur 4: in perspektivischer Darstellung einen Abschnitt eines Saugkerns,
- Figur 5: eine mögliche Ausführungsform der Erweiterung des Schlitzendes,
- Figur 6: eine weitere Ausführungsform der Erweiterung im Bereich des Schlitzendes.

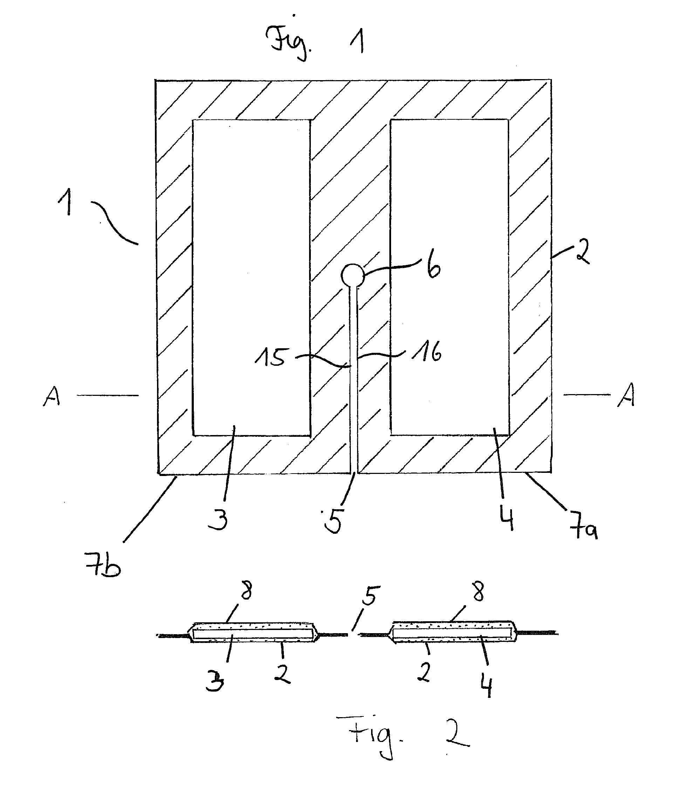

Figur 1 zeigt eine Draufsicht des erfindungsgemäßen Verbandes, der in dieser Ausgestaltung eine sogenannte Schlitzkompresse 1 darstellt. Die Schlitzkompresse weist einen Träger 2 und darauf angebrachte Saugkerne 3, 4 auf. Der Träger 2 weist einen Schlitz 5 auf, der in eine Erweiterung 6 mündet. Durch den Schlitz entsteht eine erste Trägerhälfte 7a und eine zweite Trägerhälfte 7b. Auf jeder der beiden Trägerhälften 7a, 7b sind in paralleler Ausrichtung zum Schlitz jeweils flächige Saugkerne 3, 4 angebracht.

Der erfindungsgemäße Verband kann auf Wunden oder chirurgisch bedingte Körperöffnungen aufgelegt werden, wobei ein Schlauch, eine Kanüle oder die sonstige Leitung, die aus der Wunde oder Körperöffnung hinaustritt, durch die Erweiterung 6 des Schlitzes hindurchtritt und vom Rand dieser Erweiterung umschlossen wird. Dadurch liegen auch die Ränder 15, 16 des Schlitzes der Hautoberfläche an. Aus der Körperöffnung oder Wunde heraustretende Körperflüssigkeit, wie Blut, Wundsekret oder andere Körperflüssigkeiten, kurz Exsudat genannt, wird über den Träger 2 aufgenommen und in Richtung Saugkern 3, 4 transportiert. Der Saugkern absorbiert die Flüssigkeit und speichert diese, sodass ein Rücknässen in Richtung Körperoberfläche oder auch nach außen weitgehend vermieden werden kann.

In Figur 2 ist eine Ausführungsform des erfindungsgemäßen Verbandes in der Schnittdarstellung entlang der Linie A-A dargestellt. In dieser Ausführungsform sind die Saugkerne 3 und 4 auf ihrer jeweiligen Flachseite von einem ersten Träger 2 und einem weiteren Träger 8 bedeckt. Der Träger 2 und der weitere Träger 8 weisen weitgehend die gleichen Abmessungen auf. In den Bereichen, in denen die Trägermaterialien über den jeweiligen Saugkern 3, 4 hinausragen, sind die Träger 2, 8 miteinander verbunden, vorzugsweise verklebt. In der hier dargestellten Ausführungsform sind die Saugkerne jeweils adhäsiv, vorzugsweise durch Klebstoff (hier durch Punkte dargestellt), mit dem Träger verbunden. Durch diese unmittelbare Verbindung von Träger und Saugkern erhöht sich die Stabilität des erfindungsgemäßen Verbandes und auch die Aufnahme der austretenden Körperflüssigkeit wird verbessert.

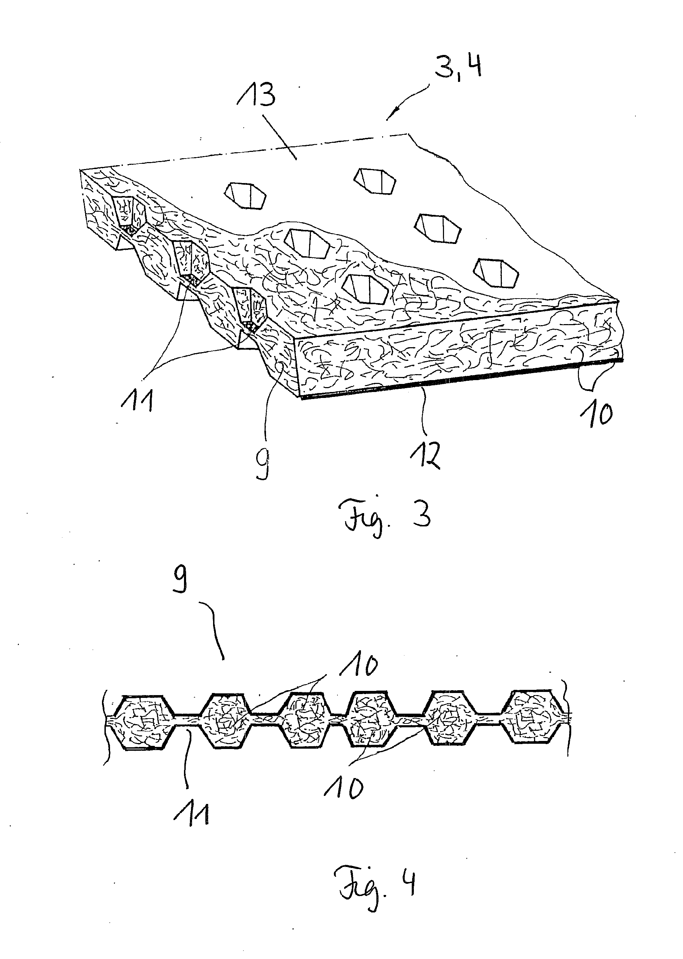

In den Figuren 3 ist in vergrößerter Darstellung eine mögliche Ausgestaltung der Saugkörpers 3, 4 im Querschnitt und in Figur 4 eine perspektivische Darstellung eines Abschnitts eines Saugkörpers 3, 4 dargestellt.

Die das Absorptionsverhalten für Flüssigkeiten, insbesondere Wundflüssigkeiten, bestimmende Schicht 9 besteht vorzugsweise aus Zellstofffasern 10. Die Zellstofffasern 10 sind in den Prägebereichen 11 verdichtet und auf diese Weise miteinander verbunden. In der hier dargestellten Ausführungsform liegen die Prägebereiche 11 sich auf der Ober- und Unterseite einander gegenüber, sodass im jeweiligen Prägebereich 11 ein nur noch schmaler Steg miteinander verbundener Zellstoffmasse verbleibt. Die übrigen, zwischen den Prägebereichen 11 angeordneten Bereiche der Schicht 9 weisen eine lockere Zellstoffschichtung auf. Eine innige Verbindung zwischen den Zellstofffasern 10 besteht in diesen Bereichen nicht. Die Prägebereiche 11 haben in der hier dargestellten Ausführungsform die Gestalt von Pyramidenstümpfen oder Kegelstümpfen, wobei der Winkel der hierdurch gebildeten Schrägen zwischen 10° und 45° liegen sollte.

Als Zellulosematerial für die Schicht 9 lässt sich preiswert zur Verfügung stehendes Massenmaterial einsetzen. Vorzugsweise wird ein möglichst ungebleichter sogenannter "fluff pulp" (auch wood pulp genannt) eingesetzt, der sich, anders als gebleichte Zellstoffe, durch ein sehr gutes Bindungsverhalten auszeichnet, was die mechanische Festigkeit des Saugkörpers gegen vertikale Zugkräfte verbessert.

Bei der Herstellung in einem kontinuierlichen Verfahren wird die später die Lage 9 bildende Faserstoffbahn aus einer im Luftstrom aufgeschichteten Schüttung von Zellstoffasern aus defiberisiertem Zellstoff (wood pulp) und ggf. eingestreuten Absorbermaterialien hergestellt. Die Faserstoffbahn aus Zellstofffasern wird durch schichtweises Schütten der Zellstofffasern erhalten. Werden weitere Absorbermaterialien, wie SAP, zugesetzt, so kann auch dieser Zusatz in einer schichtweisen Schüttung erfolgen, beispielsweise durch eine wechselnde Schüttung aus Zellstofffasern, Absorbermaterial, Zellstofffasern, so lange, bis die gewünschte Menge an Zellstoffasern und gegebenenfalls Absorbermaterial zugeführt wurde. In der in Figur 4 dargestellten Ausführungsform für einen Saugkörper weist die Schicht 9 aus Zellstofffasern 10 an ihrer Ober- und/oder Unterseite jeweils eine weitere Lage 12, 13 auf. In der hier dargestellten Ausführungsform stellt die Lage 12 die Basisschicht und die Lage 13 die Deckschicht dar. Diese Lagen 12, 13 sind vorzugsweise aus einem dünnen TissueMaterial. Dieses Material wird bereits bei der Herstellung des erfindungsgemäß verwendeten Saugkerns mit eingearbeitet, indem die Zellstoffschüttung auf das Tissue 12 aufgebracht wird und mit einem weiteren Tissue 13 abgedeckt wird. Die Schichtung aus Tissue und Zellstoff sowie bei einer gegebenenfalls weiteren Tissueschicht kann gemeinsam in den oben beschriebenen Kalander eingeführt werden. Das Tissuematerial ist im Stand der Technik bekannt und kann im Handel als Bahnmaterial erhalten werden. Das Tissuematerial gibt dem Saugkörper 6 eine weitere Stabilität, es kann auch verhindern, dass feinteiliger Abrieb der Zellstoffasern und/oder der Absorbermaterialien durch die für Flüssigkeiten durchlässige Lage A in Richtung Wunde gelangt.

Für die Herstellung einer standardisierten, defiberisierten Ware kann auf die am Markt verfügbaren, nachwachsenden Holzrohstoffe zurückgegriffen werden.

Das Verfahren der Zellstoffschüttung als Ausgangsprodukt für die Lage 9 ermöglicht eine trockene Verarbeitung der Zellstoffasern 10 und damit bei der anschließenden Prägung zwischen zwei Strukturwalzen eine sehr gute Fusionierung der Zellstoffasern in den diskreten Prägebereichen. Außerhalb der Prägebereiche 11 liegen die Fasern locker aneinander, was die Saugfähigkeit und die Flexibilität der Lage 9 verbessert.

Das Saugverhalten der Lage 9 ist im wesentlichen "trocken", d. h. solange eine vollständige Sättigung noch nicht erreicht ist, lässt sich die absorbierte Schmutzflüssigkeit nicht nach Art eines Schwammes wieder ausquetschen. Auch nach Gebrauch und solange das Absorptionsverhalten der Schicht nicht erschöpft ist, erscheint daher die Saugmatte trocken.

Die Herstellung erfolgt aus Bahnmaterial, welches in einem kontinuierlichen Prozess gefertigt wird. In luftunterstützter Schichtung werden zunächst die Zellstoffasern und die SAP's zur Bildung der Lage 9 gelegt. Anschließend erfolgt in einem Kalander mit zwei strukturierten Kalanderwalzen die Herstellung der Prägebereiche 11. In weiteren kontinuierlichen Schritten werden oben die Deckschicht 3 und unten die Basisschicht 1 angeordnet und mit der Kernschicht 9 verklebt. Das so hergestellte Bahnmaterial wird schließlich in einzelne, vorzugsweise rechteckige Felder geschnitten, die in einem weiteren Fertigungsschritt zur Herstellung des erfindungsgemäßen Absorptionskörpers zugeführt werden.

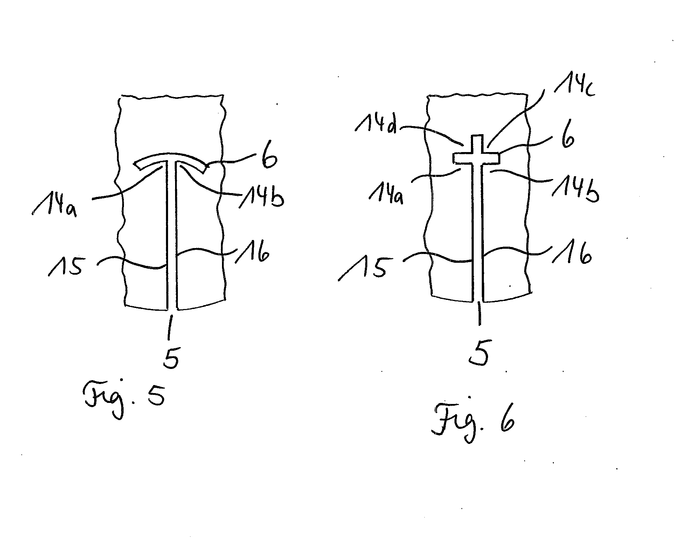

In den Figuren 5 und 6 sind zwei weitere mögliche Ausführungsformen für die Gestaltung des Schlitzes im Bereich seines Endes dargestellt.

In Figur 5 ist die Erweiterung 6 im Bereich des Endes des Schlitzes 5 ein Querschlitz, der kurz vor dem Ende des Schlitzes 5 quer zum Hauptschlitz angeordnet ist. Der Verband kann über den Schlitz 5 um die Kanüle oder den Schlauch herumgelegt werden. Die durch den Querschlitz 6 gebildeten Ecken 14a, b, c, d des Verbandmaterials können sich nach oben oder unten wegbiegen, und legen sich so an den Schlauch oder die Kanüle an, wodurch dieser bzw. diese gehalten wird. Die von dem Schlitz 5 und dem Querschlitz 6 gebildeten Ecken 14a, b, c, d biegen sich in einem solchen Maße nach oben bzw. unten, wie es die Größe des Schlauchs oder der Kanüle erfordert.

In der hier dargestellten Ausgestaltung kann der erfindungsgemäße Verband als Schlitzkompresse zur Umlage um chirurgisch gelegte Leitungen unterschiedlicher Größe eingesetzt werden.

Darüber hinaus wurde festgestellt, dass, wenn die Schichten 12, 13 aus einem für Körperflüssigkeiten durchlässigen Material sind, aus der Körperöffnung austretende Flüssigkeit bereits von dem Material der Ecken 14a, b, c, d und daran angrenzenden Bereichen aufgenommen und über die Trägerschichten in Richtung Saugkern 3, 4 transportiert werden.

In der in Figur 6 dargestellten Ausführungsform mündet die Erweiterung 6 ganz am Ende des Schlitzes 5 in einen Schlitz 6a, der im wesentlichen quer zum Hauptschlitz angeordnet ist. In der hier dargestellten Ausführungsform beschreibt dieser Querschlitz 6A einen Bogen. Der Bogen 6A bildet mit dem Ende des Schlitzes 5 die Ecken 14a, b, die sich, wie für die Ausführungsform in Figur 5 beschrieben, beiseite biegen und so an den Schlauch oder die Kanüle anlegen und diese halten können.

Der in den Figuren 5 und 6 dargestellte Schlitz 5 und die Erweiterung 6 im Bereich des Endes des Schlitzes können einfache Schnitte sein. Es ist auch möglich, den Schlitz 5 und die Erweiterung 6 etwas größer zu gestalten, indem Material in der Form des Schlitzes ausgestanzt wird, sodass die Schlitzkanten 15, 16 und die jeweils gegenüberliegenden Ecken 14a, b, c, d voneinander beabstandet sind. Ist der Schlitz ein einfacher Schnitt, stoßen die Kanten 15, 16 und die gegenüberliegenden Ecken 14a, b, c, d direkt aneinander.

### Bezugszeichenliste

- 1: Schlitzkompresse
- 2: erster Träger
- 3, 4: Saugmatte
- 5: Schlitz
- 6: Erweiterung
- 7a: erste Trägerhälfte
- 7b: zweite Trägerhälfte
- 8: weiterer Träger
- 9: Absorptionsschicht
- 10: Zellstofffasern
- 11: Prägebereich
- 12: Basisschicht
- 13: Deckschicht
- 14a, b, c, d: Ecken
- 15, 16: Schlitzkanten

## Patentansprüche

1. Verband zum Abdecken einer Körperöffnung, aus welcher ein nicht-körpereigener Strang, Schlauch, Leitung oder dgl. herausragt, welcher
einen Träger (8) mit einem vom Rand des Trägers (8) ausgehenden und in den Träger (8) hineinragenden Schlitz (5) unter Bildung einer ersten Trägerhälfte (7a) zur einen und einer zweiten Trägerhälfte (7b) zur anderen Seite des Schlitzes (5), wobei auf jeder der beiden Trägerhälften (7a, 7b) in paralleler Ausrichtung zum Schlitz (5) jeweils ein flächiger Saugkern (3, 4) angebracht ist,
**dadurch gekennzeichnet, dass**
der Schlitz (5) im Bereich seines Endes mit einer Erweiterung (6) versehen ist.

2. Verband nach Anspruch 1, **dadurch gekennzeichnet, dass** die Erweiterung (6) eine Schlüsselloch-Erweiterung ist.

3. Verband nach Anspruch 1, **dadurch gekennzeichnet, dass** die Erweiterung (6) durch Querschlitze gebildet wird.

4. Verband nach einem der Ansprüche 1 bis 3, **dadurch gekennzeichnet, dass** der flächige Saugkern (3, 4) auf seiner dem Träger (8) abgewandten Flachseite einen weiteren Träger aufweist, der die gleiche Form aufweist wie der erste Träger.

5. Verband nach Anspruch 4, **dadurch gekennzeichnet, dass** einer oder beide Träger sich über die Fläche des Saugkerns (3, 4) hinaus erstrecken und Überstände miteinander verbunden sind.

6. Verband nach einem der Ansprüche 1 bis 5, **dadurch gekennzeichnet, dass** der Saugkern (3, 4) mit dem bzw. den Träger(n) adhäsiv verbunden ist.

7. Verband nach einem der Ansprüche 1 bis 6, **dadurch gekennzeichnet, dass** der Saugkern (3, 4) ausgewählt ist aus vliesartigem oder gewebeartigem Material auf Kunststoff- oder Zellulosebasis.

8. Verband nach Anspruch 7, **dadurch gekennzeichnet, dass** der Saugkern (3, 4) ein Airlaid-Vlies ist.

9. Absorptionskörper nach einem der Ansprüche 1 bis 8, **dadurch gekennzeichnet, dass** der Saugkern (3, 4) aus Zellstoffasern mit über die Fläche des Saugkerns verteilten Prägebereichen (11) versehen ist, in denen die Zellstofffasern stärker als in den übrigen Bereichen miteinander verpresst und hierdurch klebstoff- und/oder bindemittelfrei verbunden sind.

10. Absorptionskörper nach Anspruch 9, **dadurch gekennzeichnet, dass** die Prägebereiche (11) kegelstumpf- oder pyramidenstumpfförmig sind.

11. Absorptionskörper nach einem der Ansprüche 9 oder 10, **dadurch gekennzeichnet, dass** der Saugkern (3, 4) als absorbierende Schicht aus im Luftstrom aufgeschichtete Fasern aus defiberisiertem Zellstoff (wood pulp) enthält und dass oberhalb und unterhalb dieser Fasern eine Lage aus Tissue angeordnet ist.

12. Verband nach einem der Ansprüche 1 bis 10, **dadurch gekennzeichnet, dass** der Saugkern (3, 4) Teilchen aus superabsorbierenden Polymeren enthält.

13. Verband nach einem der Ansprüche 1 bis 11, **dadurch gekennzeichnet, dass** der bzw. die Träger gleich oder verschieden sind und flüssigkeitsdurchlässig sind und ausgewählt sind aus Nonwoven, Gewebe und/oder gelochter zwei- oder dreidimensionaler Folie.

14. Verband nach einem der Ansprüche 1 bis 12, **dadurch gekennzeichnet, dass** der Saugkern (3, 4) mit dem ersten und/oder dem weiteren Träger verklebt ist.

15. Verband nach einem der Ansprüche 1 bis 14, **dadurch gekennzeichnet, dass** er eine Schlitzkompresse ist.
